# EUROPEAN PATENT SPECIFICATION

(11) **EP 3 782 590 B1**
(45) Date of publication and mention of the grant of the patent: **23.04.2025**
(21) Application number: 20187125.8
(22) Date of filing: 22.07.2020
(51) Int. Cl.: A61F 5/01

(54) **JOINT FOR HINGED BRACES**
GELENK FÜR GELENKIGE STÜTZEN
ARTICULATION POUR LES ORTHÈSES ARTICULÉE

(30) Priority: 20.08.2019 IT 201900014856
(43) Date of publication of application: 24.02.2021
(73) Proprietor: Tenortho S.R.L., 20853 Biassono (Monza Brianza) (IT)
(72) Inventor: TENTORIO, Alessio, 20052 Monza (Monza Brianza) (IT)
(74) Representative: Tansini, Elio Fabrizio

(56) References cited:
- EP-A1- 0 016 268
- CN-U- 205 698 154
- DE-U1- 202011 102 331
- DE-U1- 9 303 075
- IT-A1- UB20 152 729
- US-A- 5 772 618
- US-A1- 2017 340 471
- US-A1- 2019 142 614

## Description

The present invention relates to a joint for articular braces according to claim 1 and finds particular use in the rehabilitation field for the support and control of the flexion and/or extension of joints such as knees, elbows, etc.

The present invention also relates to a kit for supporting and controlling flexion and/or extension of an articulation joint according to claim 11.

As is known, such articular brace joints are used to "accompany" and limit the flexion and/or extension movement of the articulation within a predetermined maximum angular value. US 2017/340471 A1 discloses a joint according to the preamble of claim 1.

Generally, joints for articular braces comprise two movable rods arranged along an upper portion and a lower portion of an articulation (e.g., in the case of the elbow articulation, along the arm and along the forearm) mutually hinged to a main body containing the gears which allow the movement of the rods themselves and one or more limiting elements.

These limiting elements are inserted inside the main body of the joint in order to limit the movement of the gears and consequently the movement of the two rods which accompany the flexion and/or extension of the articulation.

When a limiting element is inserted, the rotation of the rods, and thus also the flexion and/or extension movement of the articulation, is limited to assuming only values between zero and a certain maximum value.

In particular, the flexion and/or extension movement is limited to a maximum flexion and/or extension position which varies depending on the type of limiting element inserted.

As is known, there are different limiting elements capable, depending on the shape and size thereof, of varying the aforementioned maximum value so as to allow a more or less wide extension and/or flexion of the articulation.

As is known, there are also joints for articular braces in which pins are used to control the maximum extension and/or flexion values of the articulation.

In this case, in place of the limiting elements, pins are inserted in appropriate seats of the main body. These pins allow the flexion and/or extension of the articulation to be limited to a certain maximum value.

The types of joints for articulations described above have some disadvantages.

The joints adjustable by limiting elements require a partial disassembly of the main body to allow the insertion of a limiting element.

In addition, in the case of joints arranged inside the fabrics which form the brace, it is also necessary to remove the joint in order to disassemble it and insert the respective limiting element.

For example, if the flexion of an articulation is to be allowed up to a maximum of 30°, the main body of the joint must be disassembled, the corresponding limiting element inserted and the main body reassembled.

If subsequently a maximum flexion of 45° is desired, it is again necessary to disassemble the main body of the joint to remove the first limiting element and insert the second.

The disassembly and assembly operations are therefore laborious in terms of difficulty and are time consuming.

Furthermore, considering that these operations must be carried out by a specialised technician using a specific kit, they are not actuatable by the user wearing the brace.

The joints adjustable by pins are particularly heavy, as the main body is thick and bulky. A further disadvantage of this type of joint is that each rod is adjusted separately from the other by at least two pins each, which contributes to weighing down and complicating the entire joint.

This type of joint is also delicate, as the continuous movement of the pins accentuates problems such as wear or the risk of a pin breaking inside a seat.

The technical task of the present invention is therefore to provide a joint for articular braces and a kit for supporting and controlling flexion and/or extension of an articulation joint which are able to overcome the drawbacks arising from prior art.

The object of the present invention is therefore to provide a joint for articular braces which is also easy to use by people without specific skills, such as the end user.

A further object of the present invention is to provide a joint for articular braces and kit for supporting and controlling flexion and/or extension of an articulation joint in which the process of inserting and removing the limiting elements does not require the aid of specific tools, as there is no need to disassemble the main body of the joint.

A further object of the present invention is to provide a joint for articular braces which is resistant but not heavy and bulky.

A further object of the present invention is to provide a joint for articular braces whose rods can be simultaneously adjusted easily and quickly.

A further object of the present invention is to provide a kit for supporting and controlling flexion and/or extension of an articulation joint which allows for a high versatility and ease of use, also by an inexperienced user.

The specified technical task and the objects stated are substantially achieved by a joint for articular braces and kit for supporting and controlling flexion and/or extension of an articulation joint comprising the technical features set out in one or more of the appended claims.

The dependent claims correspond to possible embodiments of the invention.

Such description will be set out hereinafter with reference to the accompanying drawings given only for illustrative and, therefore, nonlimiting purpose, wherein:
- Figure 1 shows a front view of a joint for articular braces;
- Figure 2 shows the positioning of a joint for articular braces on one knee;
- Figure 3 shows a perspective view of a joint for articular braces;
- Figures 4A and 4B show two sectional views of a joint for articular braces;
- Figure 5 shows a front view of particular elements of a joint for braces;
- Figure 6 shows a detail of a joint for articular braces.

With reference to the attached figures, "S" globally indicates a joint for articular braces.

The joint "S" for articular braces comprises a main body 1 having a first and a second end "A" and "B", opposite each other with respect to an axis of symmetry "C" of the main body 1, and a first and a second movable rod 2 and 3.

The first movable rod 2 is hinged to the first end "A" of the main body 1 while the second movable rod 3 is hinged to the second end "B" of the main body 1, so that the first and second movable rods 2 and 3 are engaged with each other to define a relative movement of mutual pivot towards/pivot away of the rods 2 and 3 themselves.

Preferably, the main body 1 comprises first and second plates 1A and 1B opposite each other so that the two movable rods 2 and 3 are engaged with each other in an area between the two plates 1A and 1B.

Preferably, the main body 1 further has a first portion "D" and a second portion "E" opposite each other with respect to an axis "F" perpendicular to the axis of symmetry "C".

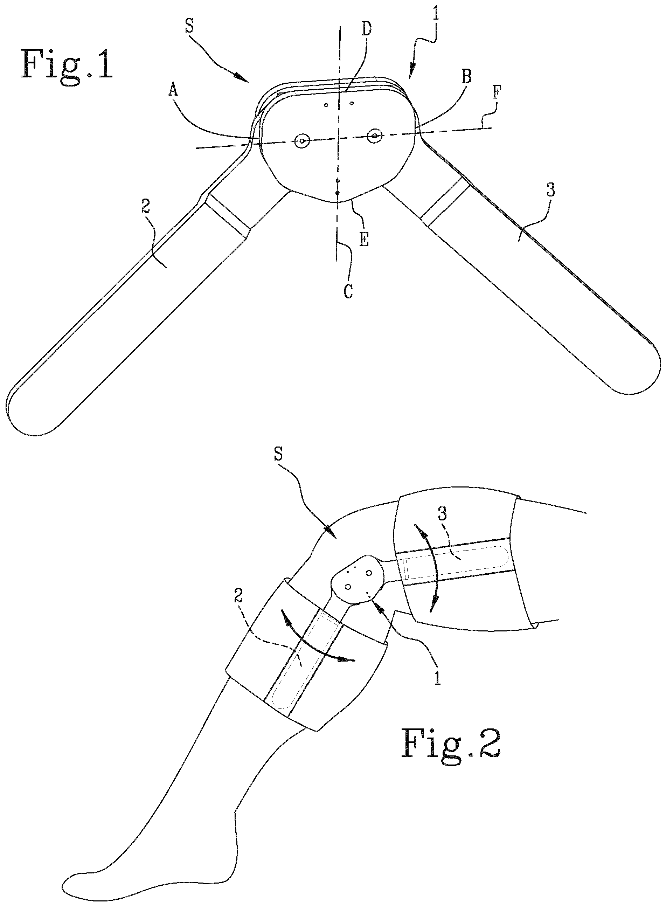

As illustrated for example in figure 2, the joint "S" for articular braces may be worn such that the main body 1 comes into contact with a knee by positioning itself laterally thereto.

In doing so, the first movable rod 2 extends along the lower part of the articulation, in the figure represented by a leg, while the second movable rod 3 extends along the upper part of the articulation, represented by a thigh.

In the case of an embodiment not shown, the main body 1 is positioned laterally to an elbow while the first movable rod 2 extends along a forearm and the second movable rod 3 extends along an arm.

In a further possible embodiment there are two "S" joints for articular braces each arranged on opposite sides of the knee/elbow.

In each of the aforementioned possible forms of use, the joint "S" for articular braces can be fixed to the articulation by the use of soft and elastic fabric frames (an example of a frame is shown in figure 2 with dashed lines).

Thanks to the support provided to the articulation by the first and second movable rods 2 and 3, said articulation is capable of moving according to an extension and/or flexion movement in a gradual and controlled manner. In other words, by the relative movement of mutual pivoting towards and/or pivoting away the two movable rods 2 and 3, the articulation may flex and/or extend according to the direction indicated by the arrows in figure 2.

In particular, the relative pivoting towards movement of the two movable rods 2 and 3 corresponds to a flexion movement of the articulation, while the relative pivoting away movement of the two movable rods 2 and 3 corresponds to an extension movement of the articulation.

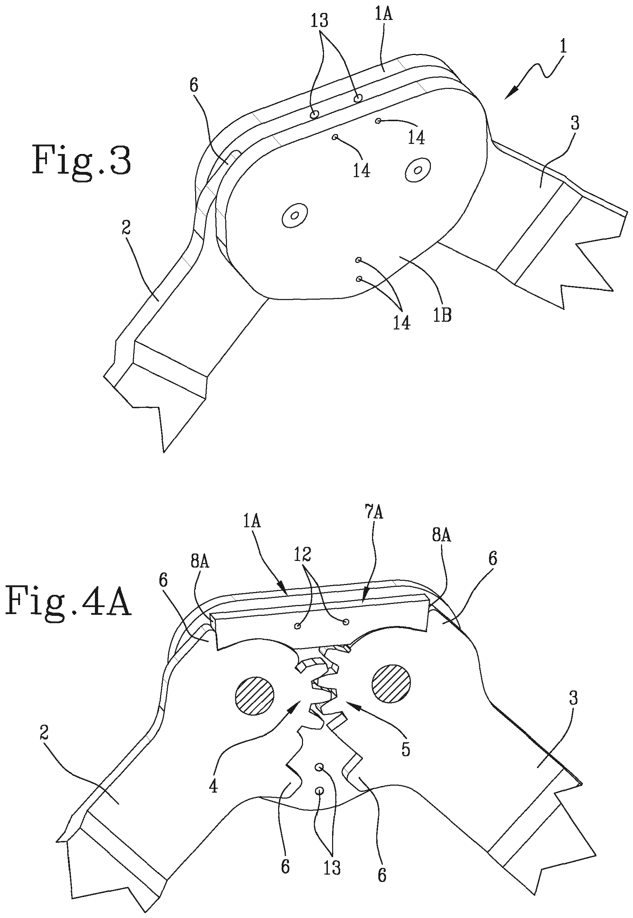

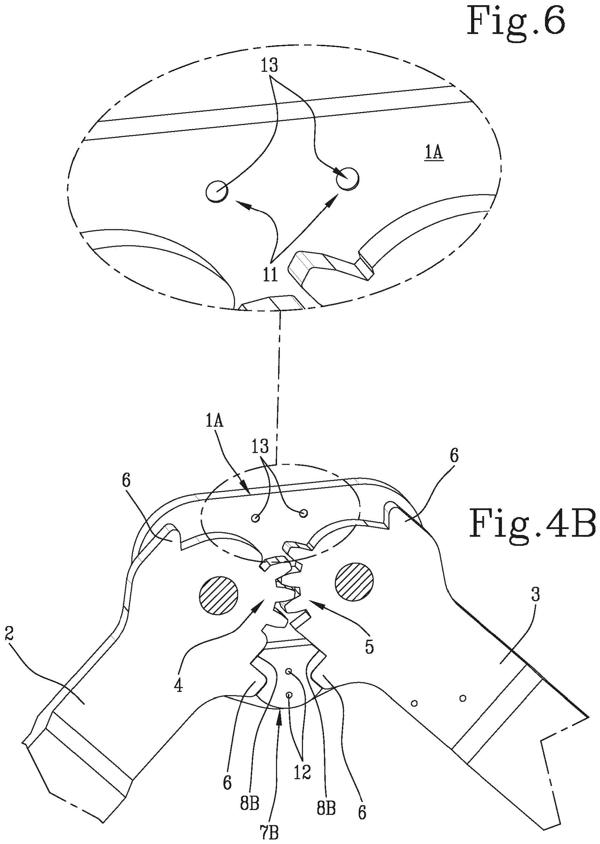

Preferably, the relative movement of mutual pivoting towards and/or pivoting away of said first and second movable rods 2 and 3 is by means of a toothed gear between the two rods. Specifically, as shown in figures 4A and 4B, the two rods 2 and 3 are engaged by a first and a second toothed sector 4 and 5.

These toothed sectors 4 and 5 are respectively provided with two recessed seats 6 arranged at the stroke ends of each toothed sector 4 and 5.

In a further embodiment not illustrated, the relative movement of mutual pivoting towards and/or pivoting away of the first and second movable rods 2 and 3 takes place thanks to two disc half-gears engaged by friction also each provided with two recessed seats 6.

The joint "S" for articular braces further comprises at least one limiting element 7 insertable between the first movable rod 2 and the second movable rod 3 to limit the relative movement thereof of mutual pivoting towards and/or pivoting away.

This limiting element 7 defines a predefined limit pivoting away position and/or a predefined limit pivoting towards position between the first and second movable rods 2 and 3 corresponding respectively to a predefined limit extension position and a predefined limit flexion position of the articulation.

In other words, thanks to the insertion of the movable element 7 between the first and second movable rods 2 and 3, the mutual movement of pivoting away and/or pivoting towards the two rods 2 and 3 is limited, consequently also the movement of extending and/or flexing the articulation is limited. The articulation can thus be extended and/or flexed only to a certain limit position measured in degrees.

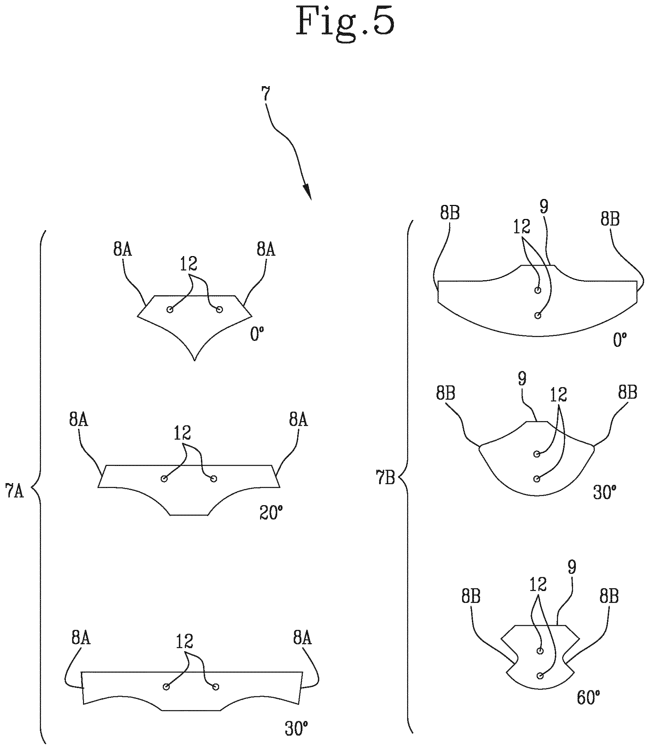

As shown in figure 5, several limiting elements 7, having different shape and size, can be inserted inside the main body 1, in this way, depending on the shape and size thereof, the precise limit position of extension and/or flexion to be obtained is determined. The limiting element 7 substantially determines the end stroke of the rotational movement of the first and the second geared sectors 4 and 5, thus identifying the position of the pivoting away/ pivoting towards limit of the two moveable rods 2 and 3.

In particular, in figure 4A, a first limiting element 7A is shown adapted to define a limit pivoting away position between the first and second movable rods 2 and 3 and, therefore, adapted to also define a limit extension position of the articulation.

This first limiting element 7A has in fact respective abutment portions 8A opposite to each other with respect to the axis of symmetry "C". Such abutment portions 8A are engaged in the respective recessed seats 6 of the first and second toothed sectors 4 and 5 in such a way as to define an end stroke portion of the pivoting away movement of the two movable rods 2 and 3 and therefore of the extension movement of the articulation.

That is, when the first limiting element 7A is inserted inside the first portion "D" of the main body 1, between the first and second movable rods 2 and 3, the abutment portions 8A thereof abut with the respective recessed seats 6 of the two toothed sectors 4 and 5 in such a way as to define an end stroke of the mutual movement of the movable rods 2 and 3 and therefore of the extension movement of the articulation.

The two toothed sectors 4 and 5 will therefore be able to rotate, one geared to the other, in such a way as to cause the mutual pivoting away of the first and second movable rods 2 and 3, and therefore an extension of the articulation, until the recessed seats 6 abut with the respective abutment portions 8A of the first limiting element 7A. This will define the limit extension position beyond which the articulation cannot extend.

Figure 5 illustrates different first limiting elements 7A which can be inserted into the first portion "D" of the main body 1 so as to limit the reciprocal pivoting away motion of the rods 2 and 3 and thus limit the extension of the articulation.

For example, if the extension of the knee is to be limited to a limit extension position of 30°, the first limiting element 7A corresponding to 30° must be inserted in the first portion "D" of the main body 1; in doing so, the first and the second toothed sector 4 and 5 can rotate until the recessed seats 6 thereof abut with the respective abutment portions 8A of the first selected limiting element 7A. In this way the mutual pivoting away movement of the first and second movable rods 2 and 3 will have a limit extension position of 30°. The articulation may thus have a range of the extension movement thereof from 0° to 30°.

Preferably, such first limiting elements 7A have a shape comprising a first straight section 9, two straight sections inclined to define the abutment portions 8A and adapted to abut with the recessed seats 6 of the toothed sectors 4 and 5, and two curved sections 10 adapted to allow the rotation of the first and second toothed sectors 4 and 5.

As shown in figure 5, depending on the extent of the first straight section 9 of the first limiting element 7A, different limit extension positions may be obtained including 0° (meaning that such first limiting element does not allow the movement of the articulation), 20° and 30°.

In other words, therefore, depending on the first limiting element 7A chosen, it will be possible to have a more or less wide range in the motion of extension of the articulation..

To define a limit pivoting towards position between the first and second movable rods 2 and 3, and therefore a limit flexion position of the articulation, a second limiting element 7B is instead used.

This second limiting element 7B is inserted into the second portion "E" of the main body 1 so as to fit between the first and the second toothed sector 4 and 5 to limit the rotation thereof and therefore limit both the relative mutual pivoting towards movement of the first and the second movable rods 2 and 3 and the flexion movement of the articulation.

The second limiting element 7B also has abutment portions 8B opposite each other with respect to the axis of symmetry "C" of the main body 1 and adapted to engage with respective recessed seats 6 of the first and second toothed sectors 4 and 5.

In other words, when the second limiting element 7B is inserted inside the second portion "E" of the main body 1, between the first and second movable rods 2 and 3, the abutment portions 8B thereof abut with the respective recessed seats 6 of the two toothed sectors 4 and 5. In this way, the interaction between the abutment portions 8B and the recessed seats 6 defines an end stroke of the mutual pivoting towards motion of the movable rods 2 and 3 and therefore of the flexion movement of the articulation.

As shown in figure 4B, the two toothed sectors 4 and 5 will therefore be able to rotate, one geared to the other, in such a way as to cause the mutual pivoting towards of the first and second movable rods 2 and 3, and therefore a flexion of the articulation, until the recessed seats 6 abut with the respective abutment portions 8B of the second limiting element 7B thus defining the limit flexion position of the articulation.

Figure 5 shows different second limiting elements 7B which can be inserted into the second portion "E" of the main body 1 so as to limit the mutual pivoting towards motion of the rods 2 and 3 and thus limit the flexion of the articulation.

For example, if the flexion of the knee is to be limited to a limit approaching position of 30°, the second limiting element 7B corresponding to 30° must be inserted in the second portion "E" of the main body 1; in doing so, the first and the second toothed sector 4 and 5 can rotate until the recessed seats 6 thereof abut with the respective abutment portions 8B of the second limiting element 7B. In this way the mutual pivoting towards movement of the first and second movable rods 2 and 3, and therefore the relative flexion movement of the articulation, will have a limit approaching position of 30°.

Figure 5 shows different second limiting elements 7B capable of determining a different limit pivoting towards position, for example, second limiting elements 7B capable of limiting articulation flexion at limit pivoting towards positions ranging from 0° (corresponding to the absence of flexion) to 75° are shown.

In other words, therefore, to limit the relative mutual pivoting away and/or pivoting towards of the first and second movable rods 2 and 3, and thus to limit the movement of extension and/or flexion of an articulation, the first and second limiting elements 7A and 7B are inserted respectively inside the first portion "D" and the second portion "E" of the main body 1 of the joint "S" for articular braces.

Advantageously, the first and the second limit element 7A and 7B are inserted/removed in the space between the walls of the main body 1 without the need to disassemble the main body 1 itself.

In the joint "S" for articular braces it is possible to insert, inside the main body 1, only the first limiting element 7A to limit the articular extension or only the second limiting element 7B to limit the articular flexion. Alternatively, both limiting elements may be simultaneously inserted into the respective portions "D" and "E" of the main body 1.

In other words, thanks to the insertion of the first and/or the second limiting element 7A and 7B, the relative movement of mutual pivoting away and/or pivoting towards of the first and second movable rods 2 and 3 is limited to a limit pivoting away and/or pivoting towards position. In particular, by inserting the first and/or second limiting elements 7A and 7B the rotational motion of the first and second toothed sectors 4 and 5 is limited; they can therefore rotate until the recessed seats 6 thereof abut with the respective abutment portions 8A and 8B of the first and second abutment elements 7A and 7B defining the maximum possible excursion for the articulation in terms of extension/flexion.

The joint "S" for articular braces further comprises at least one engagement element 11 housable in a wall of the main body 1 and reversibly engageable in a respective seat 12 of the limiting element 7 so as to keep the limiting element 7 in position during the relative movement of mutual approaching/distancing of the two movable rods 2 and 3.

Preferably, the joint "S" comprises two engagement elements 11 each arranged at a respective portion "D", "E" of the main body 1.

Preferably, the joint "S" comprises a plurality of engagement elements 11 housed in the inner walls of the respective plates 1A and 1B so as to keep both the first and second limiting elements 7A, 7B in position (i.e., between the first and second rods 2 and 3).

In other words, when the first limiting element 7A and/or the second limiting element 7B are inserted into the respective portions "D" and "E" of the main body 1, they must necessarily remain in the position in which their abutment portions 8A and 8B can be correctly engaged with the respective recessed seats 6 of the first and second toothed sectors 4 and 5. To maintain the above position, both the first limiting elements 7A and the second limiting elements 7B are provided with seats 12 adapted to receive respective engagement elements 11.

This prevents the possibility that during the rotation of the first and second toothed sectors 4 and 5, the first or second limiting elements 7A,7B will twist or even exit out of the relative portion of the main body 1.

Referring to figures 4A and 4B, the first engagement elements 7A have two seats 12 arranged horizontally relative to each other while the second engagement elements 7B have two seats 12 arranged vertically relative to each other. Each seat 12 is adapted to receive respective engagement elements 11 positioned on the walls of the main body 1.

The presence of such engagement elements 11 therefore ensures the locking of the limiting elements 7, when these are inserted inside the main body 1, and their unlocking, when these are to be extracted from the main body 1.

As shown in detail in figure 6, each engagement element 11 comprises a movable body 13 placed inside a respective housing 14 formed in the first and/or second plate 1A and 1B of the main body 1.

Such movable body 13 may move between a constraint position, in which it extends at least partially inside the seat 12 of the limiting element 7, and a release position, in which the movable body 13 is retracted inside the housing 14 to allow the insertion or removal of the limiting element 7.

Preferably, such movable bodies 13 are arranged mirrored on both walls of the main body 1.

In a further embodiment not shown, the movable bodies 13 of the engagement elements 11 are arranged on only one of the two walls of the main body 1.

Preferably, the first portion "D" of the main body 1 comprises two movable bodies 13, as well as the second portion "E" of the main body 1 comprises two movable bodies 13.

In the embodiment illustrated in figures 4A and 4B, the two movable bodies 13, arranged in the first portion "D" of the main body 1, are positioned horizontally so as to be housed inside the seats 12 of the first limiting elements 7A while the two movable bodies 13, arranged in the second portion "E" of the main body 1, are positioned vertically so as to be housed inside the seats 12 of the second limiting elements 7B.

In other words, when a first and/or second limiting element 7A and 7B are inserted into the respective portions "D" and "E" of the main body 1, the seats 12 of each limiting element 7 each receive a movable body 13, thereby the limiting elements 7A and 7B maintain the position thereof. Conversely, when a first and/or second limiting element 7A and 7B are extracted from the respective portions "D" and "E" of the main body 1, the seats 12 of each limiting element see a retraction of the movable bodies 13, thereby the limiting elements 7A and 7B can be easily extracted.

Advantageously, such movable bodies 13 of the engagement elements 11 make the insertion and extraction of the limiting elements 7 easy and fast. Advantageously, these engagement elements 11 ensure that there is no need for any specific type of tool to exchange the limiting elements 7.

In the embodiment shown in the accompanying figures, in detail in figure 6, each movable body 13 of the engagement elements 11 has a substantially spherical conformation and is made of magnetic material. In doing so, the movable body 13 is magnetically attracted inside the seat 12 of the limiting element 7 so as to assume the constraint position.

In other words, when the first and/or second limiting element 7A,7B is inserted into the main body 1 of the joint "S" for articular braces, the movable bodies 13, arranged on the inner walls of the plates 1A and 1B in the release position, are attracted by the seats 12 of the limiting elements 7A, 7B, by magnetic effect, to the constraint position. The movable bodies 13 are thus inserted in the seats 12 of the limiting elements 7 in order to force the latter to maintain the correct position between the first and second movable rods 2 and 3.

When, on the other hand, the first and/or second limiting element 7A, 7B is extracted from the inside of the main body 1 of the joint "S" for articular braces, the movable bodies 13 move to the release position from the seats 12 of the limiting elements 7A,7B, facilitating the extraction thereof. The movable bodies 13 are thus retracted from the seats 12 and housed in the housings 14 so as to release the limiting elements 7 which can exit from the main body 1.

In other words, when the limiting element 7 is inserted into the main body 1, the movable bodies 13 enter the housings 14 thereof until the limiting element 7 is correctly positioned.

When the element 7 is in position, the movable bodies 13 insert themselves into the seats 12 of the limiting element 7, locking it.

When finally the limiting element 7 is removed from the main body 1, the movable bodies re-enter the housings 14 thereof so as to release themselves with respect to the seats 12 to allow the removal of the limiting element 7.

Advantageously, the motion between the constraint position and the release position of the movable bodies 13 of the engagement elements 11 ensures an easy and quick insertion/extraction of the limiting elements 7 so that even an inexperienced user can adjust the joint "S" according to the desired articulation extension/flexion.

In addition, the motion from the constraint position to the release position is advantageously controlled (i.e., can only be performed by the user), thus avoiding unwanted extractions of the limiting element 7. In other words, each engagement element 11 advantageously allows to obtain a joint "S" for articular braces which is robust, preventing any unwanted release of the limiting elements 7.

Advantageously, the constraint and release system given by the engagement elements 11 avoids having to disassemble and reassemble, even partially, the main body 1 of the joint "S".

Also described below is a kit for supporting and controlling flexion and/or extension of an articulation joint comprising a joint "S" for articular braces according to what is described above and a plurality of first limiting elements 7A and a plurality of second limiting elements 7B.

Each of the first limiting elements 7A defines a respective limit pivoting away position which corresponds to the excursion of the pivoting away movement made by the first and second movable rods 2 and 3.

Each of the second limiting elements 7B defines a respective limit pivoting towards position corresponding to the excursion of the pivoting towards movement made by the first and second movable rods 2 and 3.

The present invention achieves the proposed object, overcoming the highlighted drawbacks of the prior art.

In fact, the joint for articular braces "S" described and claimed is versatile and easy to use, also by an inexperienced user.

Advantageously, thanks to the engagement elements 11, the joint for articular braces "S" does not need specific tools for replacing the limiting elements 7, as there is no need to disassemble the main body 1.

Advantageously, the joint "S" for articular braces allows a simultaneous adjustment of both the movable rods 2 and 3.

Advantageously, the joint for articular braces "S" is robust, although it is not bulky nor heavy.

## Claims

1. Joint (S) for articular braces comprising:
- a main body (1) having a first and second end (A, B) opposite to each other with respect to an axis of symmetry (C) of said main body (1);
- a first movable rod (2) hinged to said first end (A) of the main body (1);
- a second movable rod (3) hinged to said second end (B) of the main body (1), said rods (2, 3) being engaged with each other to define a relative movement of mutual pivoting towards or pivoting away of said first and said second movable rod (2, 3);
- at least one limiting element (7) insertable between said first movable rod (2) and said second movable rod (3) to limit the relative movement of mutual pivoting towards and/or pivoting away of said rods (2, 3); said limiting element (7) defining a predefined limit pivoting away position and/or a predefined limit pivoting towards position between said first and said second movable rod (2, 3);
- at least one engagement element (11) housable in a wall of said main body (1) and reversibly engageable in a respective seat (12) of said limiting element (7) so as to maintain said limiting element (7) in position during the relative movement of mutual pivoting towards or pivoting away of said movable rods (2, 3), said engagement element (11) comprising a movable body (13) inside a respective housing (14) obtained in the wall of the main body (1) between a constraint position in which it at least partially extends inside the seat (12) of the limiting element (7), and a release position in which it is retracted inside the housing (14) to allow the insertion or extraction of the limiting element (7);
said joint (S) being **characterized in that** said movable body (13) has a substantially spherical conformation and is made of magnetic material, said movable body (13) in the respective constraint position being attracted by magnetic effect inside the seat (12) of the limiting element (7).

2. Joint (S) for articular braces according to claim 1, wherein said first and second movable rods (2, 3) are mutually engaged by means of a first and second geared sector (4, 5) respectively engaged with each other.

3. Joint (S) for articular braces according to claim 1 or 2, comprising a first limiting element (7A) defining said limit pivoting away position; said mutual pivoting away movement of the movable rods (2, 3) corresponding to an extension movement of the articulation.

4. Joint (S) for articular braces according to claim 3, wherein said first limiting element (7A) comprises respective abutment portions (8A) opposite each other with respect to said axis of symmetry (C) of the main body (1); said abutment portions (8A) engaging respective recessed seats (6) of said toothed sectors (4, 5) of the rods (2, 3) to define an end stroke portion of said extension movement.

5. Joint (S) for articular braces according to one or more of the preceding claims, comprising a second limiting element (7B) defining said limit pivoting towards position; said mutual pivoting towards movement of the movable rods (2, 3) corresponding to a flexion movement of the articulation.

6. Joint (S) for articular braces according to claim 5, wherein said second limiting element (7B) comprises respective abutment portions (8B) opposite each other with respect to said axis of symmetry (C) of the main body (1); said abutment portions (8B) engaging respective recessed seats (6) of said toothed sectors (4, 5) of the rods (2, 3) to define an end stroke portion of said flexion movement.

7. Joint (S) for articular braces according to one or more of the preceding claims, wherein said main body (1) has a first portion (D) and a second portion (E) opposite each other with respect to an axis perpendicular (F) to said axis of symmetry (C), said first portion (D) being adapted to receive a first limiting element (7A) and said second portion (E) being adapted to receive a second limiting element (7B).

8. Joint (S) for articular braces according to one or more of the preceding claims, wherein said main body (1) comprises a first and a second plate (1A, 1B) opposite each other and spaced apart: said rods (2, 3) being engaged between said first and second plates (1A, 1B).

9. Joint (S) for articular braces according to claim 7, wherein it comprises two engagement elements (11) each of which arranged at a respective portion (D, E) of the main body (1).

10. Joint (S) for articular braces according to claim 8, wherein it comprises a plurality of engagement elements (11) housed in the inner walls of the respective plates (1A, 1B).

11. Kit for supporting and controlling flexion and/or extension of an articulation joint, **characterized in that** it comprises:
- a joint (S) for articular braces according to one or more of the preceding claims;
- a plurality of first limiting elements (7A), each of which defining a respective limit pivoting away position;
- a plurality of second limiting elements (7B), each of which defining a respective limit pivoting towards position;
said limit pivoting away and limit pivoting towards positions corresponding to the excursion of the pivoting away or pivoting towards movement performed by said movable rods (2, 3).

## Patentansprüche

1. Gelenk (S) für Gelenkstützen, umfassend:
- einen Hauptkörper (1), aufweisend ein erstes und ein zweites Ende (A, B), die in Bezug auf eine Symmetrieachse (C) des Hauptkörpers (1) gegenständig zueinander angeordnet sind;
- einen ersten bewegbaren Stab (2), der am ersten Ende (A) des Hauptkörpers (1) angelenkt ist;
- einen zweiten bewegbaren Stab (3), der am zweiten Ende (B) des Hauptkörpers (1) angelenkt ist, wobei die Stäbe (2, 3) miteinander im Eingriff sind, um eine relative Bewegung des gegenseitigen Hinschwenkens oder Wegschwenkens des ersten und des bewegbaren zweiten Stabs (2, 3) zu definieren;
- mindestens ein Begrenzungselement (7), das zwischen den ersten bewegbaren Stab (2) und den zweiten bewegbaren Stab (3) einfügbar ist, um die relative Bewegung des gegenseitigen Hinschwenkens und/oder Wegschwenkens der Stäbe (2, 3) zu begrenzen, wobei das Begrenzungselement (7) eine vordefinierte Grenzwegschwenkposition und/oder eine vordefinierte Grenzhinschwenkposition zwischen dem ersten und dem zweiten bewegbaren Stab (2; 3) definiert;
- mindestens ein Eingriffselement (11), das in einer Wand des Hauptkörpers (1) untergebracht werden kann und reversierbar in einen jeweiligen Sitz (12) des Begrenzungselements (7) eingreifbar ist, sodass das Begrenzungselement (7) während der relativen Bewegung des gegenseitigen Hinschwenkens oder Wegschwenkens der bewegbaren Stäbe (2, 3) in Position gehalten wird, wobei das Eingriffselement (11) einen bewegbaren Körper (13) in einer jeweiligen Aufnahme (14), die in der Wand des Hauptkörpers (1) ausgebildet ist, zwischen einer Befestigungsposition, in der er sich mindestens teilweise im Sitz (12) des Begrenzungselements (7) erstreckt, und einer Freigabeposition, in der er in die Aufnahme (14) eingefahren ist, um das Einfügen oder Herausnehmen des Begrenzungselements (7) zu erlauben, umfasst,
wobei das Gelenk (S) **dadurch gekennzeichnet ist, dass** der bewegbare Körper (13) eine im Wesentliche kugelförmige Beschaffenheit aufweist und aus Magnetmaterial besteht, wobei der bewegbare Körper (13) in der jeweiligen Befestigungsposition durch Magnetwirkung in den Sitz (12) des Begrenzungselements (7) angezogen wird.

2. Gelenk (S) für Gelenkstützen nach Anspruch 1, wobei der erste und der zweite bewegbare Stab (2, 3) gegenseitig mittels eines ersten und eines zweiten Zahnradsektors (4, 5), die jeweils miteinander im Eingriff sind, im Eingriff sind.

3. Gelenk (S) für Gelenkstützen nach Anspruch 1 oder 2, umfassend ein erstes Begrenzungselement (7A), das die Grenzwegschwenkposition definiert, wobei die gegenseitige Wegschwenkbewegung der bewegbaren Stäbe (2, 3) einer Streckbewegung der Gelenkverbindung entspricht.

4. Gelenk (S) für Gelenkstützen nach Anspruch 3, wobei das erste Begrenzungselement (7A) jeweilige Anschlagsabschnitte (8A) umfasst, die in Bezug auf die Symmetrieachse (C) des Hauptkörpers (1) gegenständig zueinander angeordnet sind, wobei die Anschlagsabschnitte (8A) in jeweilige vertiefte Sitze (6) der Zahnsektoren (4, 5) der Stäbe (2, 3) eingreifen, um einen Endhubabschnitt der Streckbewegung zu definieren.

5. Gelenk (S) für Gelenkstützen nach einem oder mehreren der vorhergehenden Ansprüche, umfassend ein zweites Begrenzungselement (7B), das die Grenzhinschwenkposition definiert, wobei die gegenseitige Hinschwenkbewegung der bewegbaren Stäbe (2, 3) einer Beugebewegung der Gelenkverbindung entspricht.

6. Gelenk (S) für Gelenkstützen nach Anspruch 5, wobei das zweite Begrenzungselement (7B) jeweilige Anschlagsabschnitte (8B) umfasst, die in Bezug auf die Symmetrieachse (C) des Hauptkörpers (1) gegenständig zueinander angeordnet sind, wobei die Anschlagsabschnitte (8B) in jeweilige vertiefte Sitze (6) der Zahnsektoren (4, 5) der Stäbe (2, 3) eingreifen, um einen Endhubabschnitt der Beugebewegung zu definieren.

7. Gelenk (S) für Gelenkstützen nach einem oder mehreren der vorhergehenden Ansprüche, wobei der Hauptkörper (1) einen ersten Abschnitt (D) und einen zweiten Abschnitt (E) aufweist, die in Bezug auf eine Achse senkrecht (F) zur Symmetrieachse (C) gegenständig zueinander angeordnet sind, wobei der erste Abschnitt (D) dazu geeignet ist, ein erstes Begrenzungselement (7A) aufzunehmen, und der zweite Abschnitt (E) dazu geeignet ist, ein zweites Begrenzungselement (7B) aufzunehmen.

8. Gelenk (S) für Gelenkstützen nach einem oder mehreren der vorhergehenden Ansprüche, wobei der Hauptkörper (1) eine erste und eine zweite Platte (1A, 1B) umfasst, die gegenständig zueinander und beabstandet angeordnet sind, wobei die Stäbe (2, 3) zwischen der ersten und der zweiten Platte (1A, 1B) im Eingriff sind.

9. Gelenk (S) für Gelenkstützen nach Anspruch 7, wobei es zwei Eingriffselemente (11) umfasst, von denen ein jedes an einem jeweiligen Abschnitt (D, E) des Hauptkörpers (1) angeordnet ist.

10. Gelenk (S) für Gelenkstützen nach Anspruch 8, wobei es eine Vielzahl von Eingriffselementen (11) umfasst, die in den Innenwänden der jeweiligen Platten (1A, 1B) untergebracht sind.

11. Satz zum Stützen und Steuern des Beugens und/oder Streckens eines Gelenkverbindungsgelenks, **dadurch gekennzeichnet, dass** er Folgendes umfasst:
- ein Gelenk (S) für Gelenkstützen nach einem oder mehreren der vorhergehenden Ansprüche;
- eine Vielzahl von ersten Begrenzungselementen (7A), von denen ein jedes eine jeweilige Grenzwegschwenkposition definiert;
- eine Vielzahl von zweiten Begrenzungselementen (7B), von denen ein jedes eine jeweilige Grenzhinschwenkposition definiert,
wobei die Grenzwegschwenk- und die Grenzhinschwenkpositionen der Auslenkung der Wegschwenk- oder Hinschwenkbewegung entsprechen, die von den bewegbaren Stäben (2, 3) durchgeführt wird.

## Revendications

1. Articulation (S) pour les orthèses articulées, comprenant :
- un corps principal (1) ayant une première et une deuxième extrémité (A, B) opposées l'une à l'autre par rapport à un axe de symétrie (C) dudit corps principal (1) ;
- une première tige mobile (2) articulée à ladite première extrémité (A) du corps principal (1) ;
- une seconde tige mobile (3) articulée à ladite seconde extrémité (B) du corps principal (1), lesdites tiges (2, 3) étant mises en prise l'une dans l'autre pour définir un mouvement relatif de pivotement mutuel en approche ou de pivotement en éloignement de ladite première et de ladite seconde tige mobile (2, 3) ;
- au moins un élément de limitation (7) insérable entre ladite première tige mobile (2) et ladite seconde tige mobile (3) pour limiter le mouvement relatif de pivotement mutuel en approche et/ou de pivotement en éloignement desdites tiges (2, 3) ; ledit élément de limitation (7) définissant une position limite de pivotement en éloignement prédéfinie et/ou une position limite de pivotement en éloignement prédéfinie entre lesdites première et seconde tiges mobiles (2, 3) ;
- au moins un élément de mise en prise (11) pouvant être logé dans une paroi dudit corps principal (1) et mis en prise de manière réversible dans un siège respectif (12) dudit élément de limitation (7) de manière à maintenir ledit élément de limitation (7) en position pendant le mouvement relatif de pivotement mutuel en approche ou en éloignement desdites tiges mobiles (2, 3), ledit élément d'engagement (11) comprenant un corps mobile (13) à l'intérieur d'un logement respectif (14) obtenu dans la paroi du corps principal (1) entre une position de contrainte dans laquelle il s'étend au moins partiellement à l'intérieur du siège (12) de l'élément de limitation (7), et une position de libération dans laquelle il est rétracté à l'intérieur du logement (14) pour permettre l'insertion ou l'extraction de l'élément de limitation (7) ;
ladite articulation (S) étant **caractérisée en ce que** ledit corps mobile (13) a une conformation sensiblement sphérique et est fait d'un matériau magnétique, ledit corps mobile (13) dans la position de contrainte respective étant attiré par effet magnétique à l'intérieur du siège (12) de l'élément de limitation (7) .

2. Articulation (S) pour les orthèses articulées selon la revendication 1, dans lequel lesdites première et seconde tiges mobiles (2, 3) sont mutuellement mises en prise au moyen d'un premier et d'un second secteur denté (4, 5) respectivement mis en prise l'un dans l'autre.

3. Articulation (S) pour les orthèses articulées selon la revendication 1 ou 2, comprenant un premier élément de limitation (7A) définissant ladite position limite en éloignement de pivotement ; ledit mouvement en éloignement de pivotement mutuel des tiges mobiles (2, 3) correspondant à un mouvement d'extension de l'articulation.

4. Articulation (S) pour les orthèses articulées selon la revendication 3, dans lequel ledit premier élément de limitation (7A) comprend des portions de butée respectives (8A) opposées l'une à l'autre par rapport audit axe de symétrie (C) du corps principal (1) ; lesdites portions de butée (8A) se mettant en prise dans des sièges évidés respectifs (6) desdits secteurs dentés (4, 5) des tiges (2, 3) pour définir une portion de course de fin dudit mouvement d'extension.

5. Articulation (S) pour les orthèses articulées selon une ou plusieurs des revendications précédentes, comprenant un deuxième élément de limitation (7B) définissant ladite position de pivotement en approche ; ledit mouvement mutuel de pivotement en approche des tiges mobiles (2, 3) correspondant à un mouvement de flexion de l'articulation.

6. Articulation (S) pour les orthèses articulées selon la revendication 5, dans laquelle ledit second élément de limitation (7B) comprend des portions de butée respectives (8B) opposées l'une à l'autre par rapport audit axe de symétrie (C) du corps principal (1) ; lesdites portions de butée (8B) se mettant en prise dans des sièges évidés respectifs (6) desdits secteurs dentés (4, 5) des tiges (2, 3) pour définir une portion de course de fin dudit mouvement de flexion.

7. Articulation (S) pour les orthèses articulées selon une ou plusieurs des revendications précédentes, dans lequel ledit corps principal (1) a une première portion (D) et une seconde portion (E) opposées l'une à l'autre par rapport à un axe perpendiculaire (F) audit axe de symétrie (C), ladite première portion (D) étant adaptée pour recevoir un premier élément de limitation (7A) et ladite seconde portion (E) étant adaptée pour recevoir un second élément de limitation (7B).

8. Articulation (S) pour les orthèses articulées selon une ou plusieurs des revendications précédentes, dans lequel ledit corps principal (1) comprend une première et une seconde plaque (1A, 1B) opposées l'une à l'autre et espacées l'une de l'autre : lesdites tiges (2, 3) étant mises en prise entre lesdites première et seconde plaques (1A, 1B).

9. Articulation (S) pour les orthèses articulées selon la revendication 7, dans lequel il comprend deux éléments de mise en prise (11) dont chacun est disposé au niveau d'une portion respective (D, E) du corps principal (1).

10. Articulation (S) pour les orthèses articulées selon la revendication 8, dans lequel il comprend une pluralité d'éléments de mise en prise (11) logés dans les parois internes des plaques respectives (1A, 1B).

11. Kit de support et de contrôle de la flexion et/ou de l'extension d'une articulation pivot, **caractérisé en ce qu'**il comprend :
- une articulation (S) pour les orthèses articulées selon une ou plusieurs des revendications précédentes ;
- une pluralité de premiers éléments de limitation (7A), dont chacun définit une position limite respective de pivotement en éloignement ;
- une pluralité de seconds éléments de limitation (7B), dont chacun définit une position limite respective de pivotement en approche ;
lesdites positions limites de pivotement en éloignement et limites de pivotement en approche correspondant à l'excursion du mouvement de pivotement en éloignement et de pivotement en approche effectué par lesdites tiges mobiles (2, 3).
